# EUROPÄISCHE PATENTSCHRIFT

(11) **EP 1 005 471 B1**
(45) Veröffentlichungstag und Bekanntmachung des Hinweises auf die Patenterteilung: **04.09.2002**
(21) Anmeldenummer: 99929137.0
(22) Anmeldetag: 04.06.1999
(51) Int. Cl.: C07D 487/04, A61K 31/505

(54) **ANTIKONVULSIV UND ANTIALLERGISCH/ANTIASTHMATISCH WIRKENDE PYRAZOLO(3,4-D)PYRIMIDINE**
PYRAZOLO(3,4-D)PYRIMIDINES WITH ANTICONVULSIVE AND ANTIALLERGIC/ANTIASTHMATIC ACTION
PYRAZOLO(3,4-D)PYRIMIDINES A ACTION ANTICONVULSIVE ET ANTIALLERGIQUE/ANTIASTHMATIQUE

(30) Priorität: 22.06.1998 DE 19827679
(43) Veröffentlichungstag der Anmeldung: 07.06.2000
(73) Patentinhaber: Arzneimittelwerk Dresden GmbH, 01445 Radebeul (DE)
(72) Erfinder: ARNOLD, Thomas, D-01445 Radebeul (DE); LANKAU, Hans-Joachim, D-01689 Weinböhla (DE); MENZER, Manfred, D-01279 Dresden (DE); ROSTOCK, Angelika, D-01445 Radebeul (DE); TOBER, Christine, D-01689 Weinböhla (DE); UNVERFERTH, Klaus, D-01309 Dresden (DE)
(74) Vertreter: Böhm, Brigitte, Dipl.-Chem. Dr.
(86) Internationale Anmeldenummer: EP9903863
(87) Internationale Veröffentlichungsnummer: WO99067243

(56) Entgegenhaltungen:
- WO-A-88/00192
- DE-A- 19 649 460
- GATTA F ET AL: "Pyrazolo[3,4-d]pyrimidines related to lonidamine" J. HETEROCYCL. CHEM. (JHTCAD,0022152X);1989; VOL.26 (3); PP.613-18, XP002115519 Ist. Super. Sanita;Lab. Chim. Farm.; Rome; 00161; Italy (IT)
- EL HEDI JELLALI M ET AL: "Acylation and alkylation of 4-aminopyrazolo[3,4-d]pyrimidine" TETRAHEDRON (TETRAB);1975; VOL.31 (6); PP.587-91, XP002115520 Inst. Chim. Subst. Nat.;Gif-sur-Yvette; Fr.
- EISENAECHER TH ET AL: "UEBER NEUE PYRAZOLVERBINDUNGEN: 6. MITTEILUNG: PYRAZOLO 3,4 -DPYRIMIDINYLESSIGSAEUREDERIVATE" PHARMAZIE, Bd. 46, Nr. 10, 1. Oktober 1991 (1991-10-01), Seite 747/748 XP002040530 ISSN: 0031-7144 in der Anmeldung erwähnt

## Beschreibung

Die Erfindung betrifft Pyrazolo[3,4-d]pyrimidin-4(5H)-one und Pyrazolo[3,4-d]pyrimidin-4(5H)-thione und deren Tautomere, die in 2-Stellung einen Benzyl-Rest enthalten, Verfahren zu ihrer Herstellung und ihre Verwendung als Arzneimittel, insbesondere zur Behandlung sowohl von Epilepsien verschiedener Formen als auch von allergischen Erkrankungen wie Asthma bronchiale, allergische Rhinoconjunctivitis oder atopische Dermatitis.

Der Adenosinrezeptor ist als Target zur Beeinflussung von Disregulationen in verschiedenen Organsystemen (z.B. zentrales Nervensystem, Atemwege, etc.) von Bedeutung. Pyrazolo[3,4-d]pyrimidine sind aufgrund der strukturellen Ähnlichkeiten zum Adenin pharmakologisch interessante Verbindungen.
2-Benzylsubstituierte Pyrazolo[3,4-d]pyrimidin-4(5H)-thione und Tautomere sind nicht bekannt.
Pyrazolo[3,4-d]pyrimidin-4(5H)-one und Tautomere, die einen substituierten Benzylrest in 2-Position besitzen, sind ebenfalls nicht bekannt.
Bisher wurde nur 2-Benzyl-pyrazolo[3,4-d]pyrimidin-4(5H)-on beschrieben [R. Böhm, Pharmazie 1986, 41, 430; Th. Eisenächer, R. Pech, R. Böhm, Pharmazie 1991, 46, 747]. Diese Verbindung wurde durch Cyclisierung von 3-Amino-1-benzylpyrazol-4-carbonsäureethylester mit Formamid erhalten. 2-Benzylsubstituierte 3-Amino-pyrazol-4-carbonsäureester können durch Benzylierung von 3-Aminopyrazol-4-carbonsäureester gewonnen werden [S. Senda, K. Hirota, G.-N. Yang, Chem. Pharm. Bull. 1972, 20(2) 391].
Für 2-Benzyl-pyrazolo[3,4-d]pyrimidin-4(5H)-on wurde eine konkrete pharmakologische Wirkung nicht erwähnt oder nahegelegt.

Bekannte Antikonvulsiva haben zum einen den Nachteil, daß unerwünschte Nebenwirkungen, wie Neurotoxizität und Idiosynkrasien auftreten und zum anderen diese bei bestimmten Formen der Epilepsie nicht wirksam sind.
Verschiedene Formen allergischer/asthmatischer Erkrankungen, wie Asthma bronchiale sind mit den vorhandenen Arzneien ebenfalls unzureichend behandelbar.

Der Erfindung liegt deshalb die Aufgabe zugrunde, Verbindungen mit günstigen pharmakologischen Eigenschaften zur Verfügung zu stellen, die als Arzneimittel insbesondere zur Behandlung von Epilepsien und verschiedenen allergischen/asthmatischen Erkrankungen einsetzbar sind.

Entsprechend der vorliegenden Erfindung sind diese neuen Verbindungen 2-Ar(alkyl)-pyrazolo[3,4-d]pyrimidin-4(5H)-one und 2-Ar(alkyl)-pyrazolo[3,4-d]pyrimidin-4(5H)-thione der allgemeinen Formel **1** 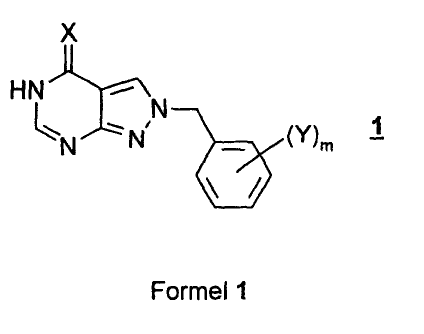 oder deren Tautomeren, wobei
- X: gleich Sauerstoff und Schwefel,
- Y: gleich Halogen, C₁-C₄-Alkyl, C₁-C₄-Alkoxy, Trifluormethyl oder Trifluormethoxy
- m: gleich 0 bis 5 bedeuten, ausgenommen 2-Benzyl-4,5-dihydro-4-oxopyrazolo [3,4-d] pyrimidin als bekanntes Zwischenprodukt.

Als Beispiele für Verbindungen der allgemeinen Formel **1** seien genannt:
2-(2-Fluorbenzyl)-pyrazolo[3,4-d]pyrimidin-4(5H)-on
2-(2-Chlorbenzyl)-pyrazolo[3,4-d]pyrimidin-4(5H)-on
2-(2-Brombenzyl)-pyrazolo[3,4-d]pyrimidin-4(5H)-on
2-(2-lodbenzyl)-pyrazolo[3,4-d]pyrimidin-4(5H)-on
2-(2-Trifluormethylbenzyl)-pyrazolo[3,4-d]pyrimidin-4(5H)-on
2-(2-Methylbenzyl)-pyrazolo[3,4-d]pyrimidin-4(5H)-on
2-(3-Trifluormethylbenzyl)-pyrazolo[3,4-d]pyrimidin-4(5H)-on
2-(2,6-Difluorbenzyl)-pyrazolo[3,4-d]pyrimidin-4(5H)-on
2-(2-Chlor-6-fluorbenzyl)-pyrazolo[3,4-d]pyrimidin-4(5H)-on
2-(2,6-Dichlorbenzyl)-pyrazolo[3,4-d]pyrimidin-4(5H)-on
2-(2,4-Dichlorbenzyl)-pyrazolo[3,4-d]pyrimidin-4(5H)-on
2-(4-Methoxybenzyl)-pyrazolo[3,4-d]pyrimidin-4(5H)-on
2-(2-Chlorbenzyl)-pyrazolo[3,4-d]pyrimidin-4(5H)-thion
2-(2-Brombenzyl)-pyrazolo[3,4-d]pyrimidin-4(5H)-thion
2-(2-lodbenzyl)-pyrazolo[3,4-d]pyrimidin-4(5H)-thion
2-(2-Trifluormethylbenzyl)-pyrazolo[3,4-d]pyrimidin-4(5H)-thion
2-(2,6-Difluorbenzyl)-pyrazolo[3,4-d]pyrimidin-4(5H)-thion

Das Verfahren zur Herstellung von Verbindungen der Formel **1** und deren Tautomere mit X gleich Sauerstoff beruht auf der Cyclisierung von 3-Aminopyrazol-4-carbonsäureestern oder 3-Aminopyrazol-4-carbonsäureamiden der allgemeinen Formel **2** in Formamid bei Temperaturen zwischen 100 und 180°C. 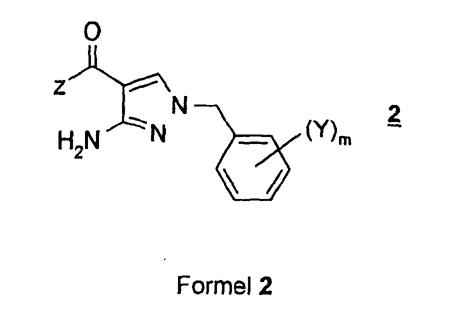 worin
Z gleich Hydroxy, Alkoxy oder Amino,
Y gleich Halogen, C₁-C₄-Alkyl, C₁-C₄-Alkoxy, Trifluormethyl, Trifluormethoxy,
m gleich 0 bis 5 bedeuten.

Das Verfahren zur Herstellung von Verbindungen der Formel **1** und deren Tautomere mit X gleich Schwefel beruht auf der Substitution von Verbindungen der Formel **1** und deren Tautomere mit X gleich Sauerstoff durch Schwefel mittels Phosphorpentasulfid oder 2,4-Bis-(4-methoxyphenyl)-1,3-dithia-2,4-diphosphetan-2,4-disulfid.

Das Verfahren zur Herstellung von Verbindungen der Formel **2** geht von 3-Aminopyrazol-4-carbonsäurederivaten aus. Durch Alkylierung unter Phasen-Transfer-Bedingungen mit einem geeignet substituierten Benzylhalogenid erhält man die Verbindungen der Formel **2.**

Die erfindungsgemäßen Verbindungen oder deren pharmazeutisch verwendbaren Salze sind zur Herstellung pharmazeutischer Zusammensetzungen geeignet. Die pharmazeutischen Zusammensetzungen beziehungsweise Medikamente können eine oder mehrere der erfindungsgemäßen Verbindungen enthalten. Zur Herstellung der pharmazeutischen Zubereitungen können die üblichen pharmazeutischen Träger- und Hilfsstoffe verwendet werden. Die Arzneimittel können beispielsweise parenteral (z. B. intravenös, intramuskulär subkutan), topisch (intranasal, inhalativ) oder oral verabreicht werden.

Die Applikationsformen können nach in der pharmazeutischen Praxis allgemein bekannten und üblichen Verfahren hergestellt werden.

Die erfindungsgemäßen Verbindungen weisen starke antikonvulsive bzw. antiallergisch/antiasthmatische Wirkungen auf.

### 1. Antikonvulsive Wirksamkeit

Die erfindungsgemäßen Verbindungen wurden in vivo nach i.p.-Applikation an Mäusen oder Ratten (p.o.-Applikation) nach dem international üblichen Standard (Pharmac.Weekblad, Sc.Ed. **14,** 132 (1992) und Antiepileptic Drugs, Third.Ed., Raven Press, New York 1989) auf ihre antikonvulsive Wirkung getestet (Tabelle 1).

Für die orale Wirkung wurden analoge Ergebnisse erhalten.
Beispielsweise wurde für die Verbindung 2 (2-(2-Chlorbenzyl)-pyrazolo[3,4-d]pyrimidin-4(5H)-on) bei der Ratte im Maximalen Elektroschock die ED₅₀ (p.o.) zu 32 mg/kg und für die Neurotoxizität die NT₅₀ > 250 mglkg bestimmt. Verbindung 14 (2-(2-Chlorbenzyl)-pyrazolo[3,4-d]pyrimidin-4(5H)-thion) ist ebenfalls stark antikonvulsiv bei grosser therapeutischer Breite (ED₅₀ (Ratte p.o.) = 12mg/kg, NT₅₀ > 460 mg/kg).

**Tabelle 1:**

| Antikonvulsive Wirkung von ausgewählten Pyrazolo[3,4-d]pyrimidinen | | | | |
|---|---|---|---|---|
| Verbindung¹⁾ | log P²⁾ | Test³⁾ | Dosis⁴⁾ | Wirkung⁵⁾ |
| | 0.2 | MES | 30 | 100 |
| 1 | | PTZ | 100 | 100 |
| | 1.01 | MES | 100 | 100 |
| 2 | | PTZ | 100 | 60 |
| | 0.56 | MES | 100 | 100 |
| 3 | | PTZ | 300 | 80 |
| | 0.74 | MES | 300 | 100 |
| 4 | | PTZ | 300 | -- |
| | 1.08 | MES | 30 | 100 |
| 5 | | PTZ | 30 | 20 |
| | 0.75 | MES | 30 | 100 |
| 6 | | PTZ | 100 | 20 |
| | 1.00 | MES | 30 | 100 |
| 7 | | PTZ | 100 | 60 |
| | 0.18 | MES | 30 | 100 |
| 8 | | PTZ | 30 | 40 |
| | 0.93 | MES | 30 | 100 |
| 9 | | PTZ | 300 | -- |
| | 1.16 | MES | 100 | 60 |
| 10 | | PTZ | 300 | -- |
| | 1.68 | MES | 100 | 100 |
| 11 | | PTZ | 100 | 20 |
| | 0.62 | MES | 100 | 15 |
| 12 | | PTZ | 300 | 40 |
| | 1.54 | MES | 100 | 100 |
| 14 | | PTZ | 100 | 60 |
| | 1.20 | MES | 100 | 30 |
| 15 | | PTZ | 300 | - |
| | 1.40 | MES | 100 | 30 |
| 16 | | PTZ | 300 | -- |
| | 1.60 | MES | 100 | 100 |
| 17 | | PTZ | 100 | 80 |
| | 1.57 | MES | 30 | 100 |
| 18 | | PTZ | 100 | 60 |
| 2-Benzylpyrazolo-[3,4-d]-pyrimidin | 0.40 | MES | 100 | 100 |
| | | PTZ | 100 | -- |
| Vergleichssubstanzen | | | | |
| Carbamazepin | | MES | 100 | 100 |
| | | PTZ | 100 | 0 |
| Valproat | | MES | 100 | 0 |
| | | PTZ | 100 | 30 |

| | | | | |
|---|---|---|---|---|
| Anmerkungen zur Tabelle 1: 1) Numerierung der Verbindungen entsprechend der Beispiele in Tabelle 2 und Tabelle 3 | | | | |
| 2) Verteilungskoeffizient Octanot/Wasser | | | | |
| 3) Maus i.p.: MES = maximaler Elektroschock, PTZ = s.c. Pentetrazol | | | | |
| 4) in mg/kg | | | | |
| 5) in % der geschützten Tiere; n.t. = fehlende Testergebnisse | | | | |

### 2. Antiallergische/antiasthmatische Wirksamkeit:

Die erfindungsgemäßen Verbindungen wurden in vivo nach oraler Applikation am männlichen Meerschweinchen auf antiasthmatische Wirkung getestet, wobei die Hemmung der Einwanderung der eosinophilen Granulozyten in die Lunge bestimmt wurde.
Männliche Meerschweinchen (Dunkin Hartley Shoe) im Gewicht von 200 - 250 g wurden aktiv sensibilisiert durch eine s.c. Injektion von Ovalbumin (10µg + 1 mg Aluminiumhydroxid) und 2 Wochen später geboostert. Ein Woche nach der Boosterung mit Ovalbumin wurden die Tiere mit einem Aerosol aus 0,5 %iger Ovalbuminlösung für 20 - 30 sec. provoziert (gechallenged). 24 h später wurde bei den Tieren in Urethannarkose eine bronchoalveoläre Lavage (BAL) mit 2 x 5 ml Kochsalzlösung durchgeführt. Die Lavageflüssigkeit wurde gesammelt und bei 400 x g für 10 min zentrifugiert und die Zellpellets in 1 ml Kochsalzlösung suspendiert. Die eosinophilen Granulozyten wurden mikroskopisch in einer Neubauerkammer gezählt. Zur Anfärbung wurde der Becton Dickinson Test kit (No. 5877) für Eosinophile verwendet. Der Test kit verwendet Phloxin B als selektive Färbung für Eosinophile. Für jedes Tier wurden die Eosinophilen in der BAL gezählt und die Eosinophilen in Mio/Tier berechnet. Die Testsubstanzen wurden 2 Stunden vor der Allergen-Provokation oral verabreicht.

Die prozentuale Hemmung der Eosinophilie der mit Substanz behandelten Gruppe wird nach folgender Formel berechnet:
(A - C) - (B - C) / (A - C) x 100 = % Hemmung
A = Eosinophile in der nicht behandelten Challenge - Kontrollgruppe
B = Eosinophile in der mit Substanz behandelten Challenge - Gruppe
C = Eosinophile in der nicht gechallenged Kontroll - Gruppe

Beispielsweise hemmt Verbindung 3 bei einer Dosis von 100 mglkg die für die Asthma bronchiale charakteristische Einwanderung der eosinophilen Granulozyten in die Lunge um 74 %.
Die erfindungsgemäßen Verbindungen sind beispielsweise zur Behandlung von Asthma bronchiale, Rhinoconjunctivits und atopische Dermatitis geeignet.

Die erfindungsgemäßen Verbindungen binden subtypspezifisch (A₃) an den Adenosinrezeptor.
Adenosin-A₃-Rezeptorantagonisten können durch die Blockade ihrer Rezeptoren die Mediatorausschüttung verhindern. Vor kurzem ist gezeigt worden, daß Adenosin-A₃-Rezeptorantagonisten die Absenkung der intracellulären cAMP-Konzentration auch in den eosinophilen Granulozyten verhindern und dadurch auch die Freisetzung von den Cytokinen und anderen Mediatoren aus diesen Zellen (Jacobson et al., 1995) unterdrücken. Dadurch können einerseits die unangenehmen, die Lebensqualität stark beeinträchtigenden akuten Symptome gelindert werden und andererseits auch die der Erkrankung unterliegenden entzündlichen Prozesse unterdrückt werden.

**Tabelle 2:**

| Kᵢ-Werte [µM] am Adenosinrezeptor (A₃)und Selektivitäten | | | |
|---|---|---|---|
| Verbindung¹⁾ | Kᵢ-Werte [µM] | A₃/A₁ | A₃/A₂ |
| 1 | 3,2 | 0,7 | 0.01 |
| 2 | 2 | 0,02 | 0,06 |
| 3 | 0,43 | 0,008 | 0.005 |
| 4 | 0,49 | 0,008 | < 0,0005 |
| 5 | 0,79 | 0,002 | 0,007 |
| 6 | 4 | 0,01 | 0,08 |
| 9 | 1 | 0,002 | 0,004 |
| 10 | 1,6 | 0,02 | 0,02 |
| 11 | 0,71 | 0,04 | 0,01 |
| 12 | 9,8 | 0,5 | 0,01 |
| 14 | 0,5 | 0,007 | < 0,001 |
| 15 | 0,34 | 0,005 | < 0,001 |
| 16 | 0,5 | 0,008 | < 0,001 |

| | | | |
|---|---|---|---|
| Anmerkungen zur Tabelle 2: 1) Numerierung der Verbindungen entsprechend der Beispiele in Tabelle 3 und Tabelle 4 | | | |

Die in der Tabelle 2 dargestellten Kᵢ-Werte zeigen auf, daß die erfindungsgemäßen Verbindungen an den Rezeptoren binden, die Selektivitätswerte (Spalte 2 und 3) zeigen, daß diese Verbindungen dabei selektiv binden. Durch diesen neuartigen Bindungsmechanismus kann möglicherweise eine biologische Wirksamkeit vermittelt werden.

Die folgenden Beispiele dienen der weiteren Erläuterung der Erfindung ohne diese zu beschränken.

### Allgemeine Vorschrift zur Herstellung der Verbindungen der Formel 1 und deren Tautomere mit X gleich Sauerstoff gemäß Tabelle 3, Beispiele 1 - 12.

Zu 40 ml Formamid gibt man 30 mmol Verbindung der Formel **2** und erhitzt zwischen 100 und 200°C für 8-16 Stunden. Nach Abkühlen wird das ausgefallene Rohprodukt abgesaugt und mit einem geeigneten Lösungsmittel z. B. Ethanol oder DMF wird eine Umkristallisation (UK) durchgeführt.

**Tabelle 3:**

| Pyrazolo[3,4-d]pyrimidine, X = O, Beispiele 1 - 12 | | | | |
|---|---|---|---|---|
| Verbindung | Y | Ausbeute (%) | Fp(°C) | Umkristallisation aus: |
| 1 | 2-F | 40 | 230-234 | EtOH |
| 2 | 2-CI | 51 | 228-231 | EtOH |
| 3 | 2-Br | 26 | 227-232 | EtOH |
| 4 | 2-1 | 28 | 265-268 | EtOH |
| 5 | 2-CF₃ | 48 | 224-226 | EtOH |
| 6 | 2-CH₃ | 65 | 262-264 | EtOH |
| 7 | 3-CF₃ | 56 | 243-244 | EtOH |
| 8 | 2,6-F₂ | 42 | 251-253 | EtOH |
| 9 | 2-Cl-6-F | 61 | 254-256 | EtOH |
| 10 | 2,6-Cl₂ | 76 | 268-271 | EtOH |
| 11 | 2,4-Cl₂ | 41 | 255-257 | EtOH |
| 12 | 4-OCH₃ | 64 | 260-261 | EtOH |

### Allgemeine Vorschrift zur Herstellung der Verbindungen der Formel 1 und deren Tautomere mit X gleich Schwefel gemäß Tabelle 4, Beispiele 13 - 19.

### Methode A:

Zu 100 ml Pyridin gibt man 20 mmol der Verbindung der Formel **1** mit X gleich Sauerstoff und 80 mmol Phosporpentasulfid und erhitzt zwischen 80 und 115°C für 4 bis 8 Stunden. Nach Abkühlen wird das ausgefallene Rohprodukt aus einem geeigneten Lösungsmittel vorzugsweise Ethanol mittels Umkristallisation (UK) gereinigt.

### Methode B:

Zu 100 ml Xylol gibt man 10 mmol der Verbindung der Formel **1** mit X gleich Sauerstoff und 20 mmol 2,4-Bis-(4-methoxyphenyl)-1,3-dithia-2,4-diphosphetan-2,4-disulfid und erhitzt 8 bis24 Stunden. Man filtriert das Rohprodukt ab und reinigt wie bei Methode A.

**Tabelle 4:**

| Pyrazolo[3,4-d]pyrimidine, X = S, Beispiele 13 - 19 | | | | | |
|---|---|---|---|---|---|
| Verbindung | Y | Ausbeute (%) | Fp (°C) | Methode | Umkristallisation aus |
| 13 | 2-F | 62 | 272-275 | A | EtOH |
| 14 | 2-Cl | 98 | 247-249 | B | DMF |
| 15 | 2-Br | 66 | 263-264 | A | EtOH |
| 16 | 2-I | 64 | 278-281 | A | DMF |
| 17 | 2-CF₃ | 93 | 303-305 | B | EtOH |
| 18 | 2,6-F₂ | 72 | 293-297 | B | EtOH |
| 19 | 2-Cl-6-F | 47 | 252-253 | A | AcOH |

## Patentansprüche

1. Neue Verbindungen der allgemeinen Formel 1 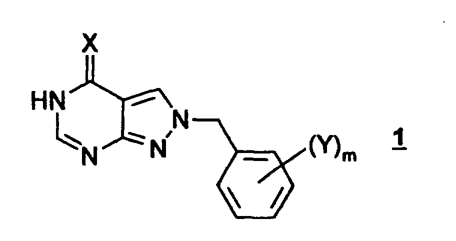 oder deren Tautomere, worin bedeuten
X gleich Sauerstoff oder Schwefel und
Y gleich Halogen, C₁-C₄-Alkyl, C₁-C₄-Alkoxy, Trifluormethyl oder Trifluormethoxy
m gleich 0 bis 5, ausgenommen die Verbindung 2-Benzyl 4,5-dihydro-4-oxopyrazolo[3,4-d]pyrimidin.

2. Verbindungen der allgemeinen Formel 1:
2-(2-Fluorbenzyl)-pyrazolo[3,4-d]pyrimidin-4(5H)-on
2-(2-Chlorbenzyl)-pyrazolo[3,4-d]pyrimidin-4(5H)-on
2-(2-Brombenzyl)-pyrazolo[3,4-d]pyrimidin-4(5H)-on
2-(2-lodbenzyl)-pyrazolo[3,4-d]pyrimidin-4(5H)-on
2-(2-Trifluormethylbenzyl)-pyrazolo[3,4-d]pyrimidin-4(5H)-on
2-(2-Methylbenzyl)-pyrazolo[3,4-d]pyrimidin-4(5H)-on
2-(3-Trifluormethylbenzyl)-pyrazolo[3,4-d]pyrimidin-4(5H)-on
2-(2,6-Difluorbenzyl)-pyrazolo[3,4-d]pyrimidin-4(5H)-on
2-(2-Chlor-6-fluorbenzyl)-pyrazolo[3,4-d]pyrimidin-4(5H)-on
2-(2,6-Dichlorbenzyl)-pyrazolo[3,4-d]pyrimidin-4(5H)-on
2-(2,4-Dichlorbenzyl)-pyrazolo[3,4-d]pyrimidin-4(5H)-on
2-(4-Methoxybenzyl)-pyrazolo[3,4-d]pyrimidin-4(5H)-on
2-(2-Chlorbenzyl)-pyrazolo[3,4-d]pyrimidin-4(5H)-thion
2-(2-Brombenzyl)-pyrazolo[3,4-d]pyrimidin-4(5H)-thion
2-(2-lodbenzyl)-pyrazolo[3,4-d]pyrimidin-4(5H)-thion
2-(2-Trifluormethylbenzyl)-pyrazolo[3,4-d]pyrimidin-4(5H)-thion
2-(2,6-Difluorbenzyl)-pyrazolo[3,4-d]pyrimidin-4(5H)-thion

3. Verfahren zur Herstellung von Pyrazolo[3,4-d]pyrimidinen der allgemeinen Formel **1** mit X = Sauerstoff **dadurch gekennzeichnet, daß** man eine Verbindung der Formel **2** mit Formamid cyclisiert 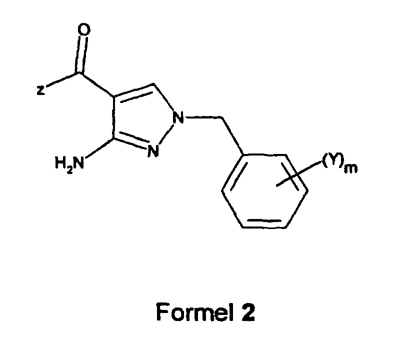 worin
Z gleich Hydroxy, Alkoxy oder Amino
Y gleich Halogen, C₁-C₄-Alkyl, C₁-C₄-Alkoxy, Trifluormethyl oder Trifluormethoxy und m gleich 0 bis 5 bedeuten.

4. Verfahren nach Anspruch 3 **dadurch gekennzeichnet, daß** zur Cyclisierung von 3-Amino-pyrazol-4-carbonsäureamiden der Formel **2** Orthoameisensäurenalkylester oder Ameisensäure/Acetanhydrid-Gemische verwendet werden.

5. Verfahren zur Herstellung von Pyrazolo[3,4-d]pyrimidinen der allgemeinen Formel **1** mit X = Schwefel **dadurch gekennzeichnet, daß** man eine Verbindung der Formel **1** mit X = Sauerstoff mit Phosphorpentasulfid oder 2,4-Bis-(4-methoxyphenyl)-1,3-dithia-2,4-diphosphetan-2,4-disulfid zur Reaktion bringt.

6. Pharmazeutische Zusammensetzungen, **gekennzeichnet durch** einen Gehalt an ein oder mehrere Pyrazolo[3,4-d]pyrimidine der Formel 1 nach Anspruch 1 oder deren pharmazeutisch verwendbaren Salze als Wirkstoffe neben einem oder mehreren physiologisch verträglichen Hilfs- und/oder Trägerstoffen und gegebenenfalls einem Verdünnungsmittel.

7. Pharmazeutische Zusammensetzungen, **gekennzeichnet durch** einen Gehalt an ein oder mehrere Pyrazolo[3,4-d]pyrimidine nach Anspruch 2 oder deren pharmazeutisch verwendbaren Salze als Wirkstoffe neben einem oder mehreren physiologisch verträglichen Hilfs- und/oder Trägerstoffen und gegebenenfalls einem Verdünnungsmittel.

8. Verwendung von Pyrazolo[3,4-d]pyrimidinen gemäß Formel **1** zur Herstellung von Arzneimitteln zur Behandlung insbesondere von Epilepsien verschiedener Formen.

9. Verwendung von Pyrazolo[3,4-d]pyrimidinen gemäß Formel **1** zur Herstellung von Arzneimitteln zur Behandlung insbesondere von allergischen/asthmatischen Erkrankungen.

## Claims

1. Novel compounds of the general formula **1** 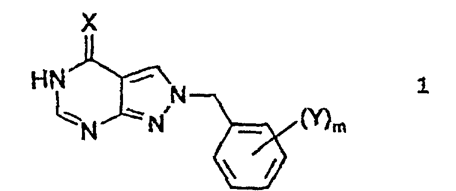 or their tautomers, wherein
X is equivalent to oxygen or sulphur and
Y is equivalent to halogen, C₁-C₄ alkyl, C₁-C₄ alkoxy, trifluoromethyl or trifluoromethoxy
m is equal to 0 to 5, excluding the compound 2-benzyl 4, 5-dihydro-4-oxopyrazolo[3,4-d]pyrimidine.

2. Compounds of the general formula **1**:
2-(2-fluorobenzyl)pyrazolo[3,4-d]pyrimidin-4(5*H*)-one,
2-(2-chlorobenzyl)pyrazolo[3,4-d]pyrimidin-4(5*H*)-one,
2-(2-bromobenzyl)pyrazolo[3,4-d]pyrimidin-4(5*H*)-one,
2-(2-iodobenzyl)pyrazolo[3,4-d]pyrimidin-4(5*H*)-one,
2-(2-trifluoromethylbenzyl)pyrazolo[3,4-d]pyrimidin-4(5*H*)-one,
2-(2-methylbenzyl)pyrazolo[3,4-d]pyrimidin-4(5*H*)-one,
2-(3-trifluoromethylbenzyl)pyrazolo[3,4-d]pyrimidin-4(5*H*)-one,
2-(2,6-difluorobenzyl)pyrazolo[3,4-d]pyrimidin-4(5*H*)-one,
2-(2-chloro-6-fluorobenzyl)pyrazolo[3,4-d]pyrimidin-4(5*H*)-one,
2-(2,6-dichlorobenzyl)pyrazolo[3,4-d]pyrimidin-4(5*H*)-one,
2-(2,4-dichlorobenzyl)pyrazolo[3,4-d]pyrimidin-4(5*H*)-one,
2-(4-methoxybenzyl)pyrazolo[3,4-d]pyrimidin-4(5*H*)-one,
2-(2-chlorobenzyl)pyrazolo[3,4-d]pyrimidine-4(5*H*)-thione,
2-(2-bromobenzyl)pyrazole[3,4-d]pyrimidine-4(5*H*)-thione,
2-(2-iodobenzyl)pyrazolo[3,4-d]pyrimidine-4(5*H*)-thione,
2-(2-trifluoromethylbenzyl)pyrazolo[3,4-d]pyrimidine-4(5*H*)-thione, and,
2-(2,6-difluorobenzyl)pyrazolo[3,4-d]pyrimidine-4(5*H*)-thione.

3. A process for the preparation of pyrazolo[3,4-d] pyrimidines of the general formula **1**, where X is oxygen, **characterised by** cyclising with formamide a compound of formula **2** 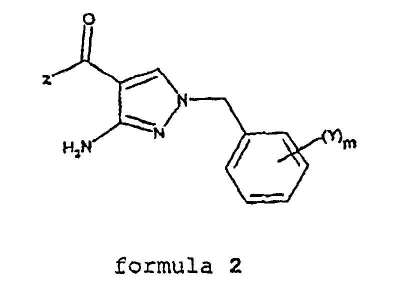 wherein
Z is equivalent to hydroxy, alkoxy or amino
Y is equivalent to halogen, C₁-C₄ alkyl, C₁-C₄ alkoxy, trifluoromethyl or trifluoromethoxy and m is equal to 0 to 5.

4. A process according to Claim 3, **characterised in that** for cyclising 3-aminopyrazole-4-carboxamides of formula **2**, alkyl orthoformate or mixtures of formic acid and acetic anhydride are used.

5. A process for the preparation of pyrazolo[3,4-d] pyrimidines of the general formula **1**, where X is sulphur, **characterised by** reacting a compound of formula **1** where X is oxygen with phosphorus pentasulphide, or 2,4-bis(4-methoxyphenyl)-1,3-dithia-2,4-diphosphetane-2,4-disulphide.

6. Pharmaceutical compositions, **characterised by** containing one or more pyrazolo[3,4-d]pyrimidines of the formula **1** according to Claim 1 or their pharmaceutically applicable salts as active ingredients, in addition to one or more physiologically compatible auxiliary agents and/or excipients, and optionally a diluent.

7. Pharmaceutical compositions, **characterised by** containing one or more pyrazolo[3,4-d]pyrimidines according to Claim 2, or their pharmaceutically applicable salts as active ingredients, in addition to one or more physiologically compatible auxiliary agents and/or excipients and, optionally a diluent.

8. Use of pyrazolo[3,4-d]pyrimidines according to formula **1** for the preparation of medicaments for the treatment, in particular, of different forms of epilepsy.

9. Use of pyrazolo[3,4-d]pyrimidines according to formula **1** for the preparation of medicaments for the treatment, in particular, of allergic/asthmatic conditions.

## Revendications

1. Nouveaux composés de formule générale **1** 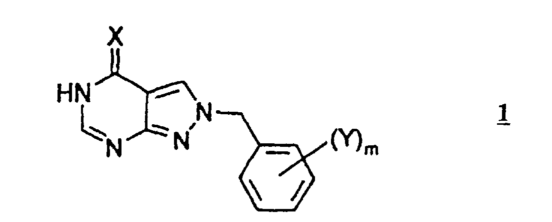 ou leurs tautomères, où
X représente l'oxygène ou le soufre et
Y représente un halogène ou un groupe alkyle en C₁-C₄, alcoxy en C₁-C₄, trifluorométhyle ou trifluorométhoxy,
m est un nombre de 0 à 5,
à l'exception du composé 2-benzyl-4,5-dihydro-4-oxopyrazolo[3,4-d]pyrimidine.

2. Composés de formule générale **1**:
2-(2-fluorobenzyl)pyrazolo[3,4-d]pyrimidin-4(5H)-one
2-(2-chlorobenzyl)pyrazolo[3,4-d]pyrimidin-4(5H)-one
2-(2-bromobenzyl)pyrazolo[3,4-d]pyrimidin-4(5H)-one
2-(2-iodobenzyl)pyrazolo[3,4-d]pyrimidin-4(5H)-one
2-(2-trifluorométhylbenzyl)pyrazolo[3,4-d]pyrimidin-4(5H)-one
2-(2-méthylbenzyl)pyrazolo[3,4-d]pyrimidin-4(5H)-one
2-(3-trifluorométhylbenzyl)pyrazolo[3,4-d]pyrimidin-4(5H)-one
2-(2,6-difluorobenzyl)pyrazolo[3,4-d]pyrimidin-4(5H)-one
2-(2-chloro-6-fluorobenzyl)pyrazolo[3,4-d]pyrimidin-4(5H)-one
2-(2,6-dichlorobenzyl)pyrazolo[3,4-d]pyrimidin-4(5H)-one
2-(2,4-dichlorobenzyl)pyrazolo[3,4-d]pyrimidin-4(5H)-one
2-(4-méthoxybenzyl)pyrazolo[3,4-d]pyrimidin-4(5H)-one
2-(2-chlorobenzyl)pyrazolo[3,4-d]pyrimidine-4(5H)-thione
2-(2-bromobenzyl)pyrazolo[3,4-d]pyrimidine-4(5H)-thione
2-(2-iodobenzyl)pyrazolo[3,4-d]pyrimidine-4(5H)-thione
2-(2-trifluorométhylbenzyl)pyrazolo[3,4-d]pyrimidine-4(5H)-thione
2-(2,6-difluorobenzyl)pyrazolo[3,4-d]pyrimidine-4(5H)-thione.

3. Procédé de préparation de pyrazolo[3,4-d]pyrimidines de formule générale **1** dans laquelle X représente l'oxygène, **caractérisé en ce que** l'on cyclise avec du formamide un composé de formule **2** 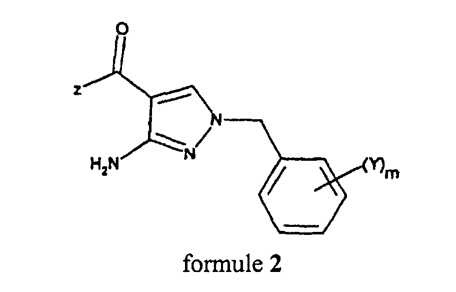 dans laquelle
Z représente un groupe hydroxy, alcoxy ou amino,
Y représente un halogène ou un groupe alkyle en C₁-C₄, alcoxy en C₁-C₄, trifluorométhyle ou trifluorométhoxy et
m est un nombre de 0 à 5.

4. Procédé selon la revendication 3, **caractérisé en ce que** l'on utilise des orthoformates d'alkyle ou des mélanges acide formique/acétanhydride pour la cyclisation de 3-aminopyrazole-4-carboxamides de formule **2**.

5. Procédé de préparation de pyrazolo[3,4-d]pyrimidines de formule générale **1** dans laquelle X représente le soufre, **caractérisé en ce que** l'on fait réagir un composé de formule **1** dans laquelle X représente l'oxygène avec du pentasulfure de phosphore ou du 2,4-bis(4-méthoxyphényl)-1,3-dithia-2,4-diphosphétane-2,4-disulfure.

6. Compositions pharmaceutiques, **caractérisées en ce qu'**elles contiennent une ou plusieurs pyrazolo[3,4-d]pyrimidines de formule **1** selon la revendication 1 ou leurs sels pharmaceutiquement acceptables comme substances actives avec un ou plusieurs additifs et/ou supports physiologiquement compatibles et éventuellement un diluant.

7. Compositions pharmaceutiques, **caractérisées en ce qu'**elles contiennent une ou plusieurs pyrazolo[3,4-d]pyrimidines selon la revendication 2 comme substances actives ou leurs sels pharmaceutiquement acceptables avec un ou plusieurs additifs et/ou supports physiologiquement compatibles et éventuellement un diluant.

8. Utilisation de pyrazolo[3,4-d]pyrimidines de formule **1** pour la préparation de médicaments destinés au traitement en particulier de différentes formes d'épilepsies.

9. Utilisation de pyrazolo[3,4-d]pyrimidines de formule **1** pour la préparation de médicaments destinés au traitement en particulier de maladies allergiques/asthmatiques.
